(19) Europäisches Patentamt
European Patent Office
Office européen des brevets

(11) EP 1 395 673 B1

(12) **EUROPÄISCHE PATENTSCHRIFT**

(45) Veröffentlichungstag und Bekanntmachung des Hinweises auf die Patenterteilung:
**05.04.2006 Patentblatt 2006/14**

(21) Anmeldenummer: **02735371.3**

(22) Anmeldetag: **21.05.2002**

(51) Int Cl.:
*C12Q 1/34* (2006.01) *C12Q 1/44* (2006.01)
*C12Q 1/37* (2006.01)

(86) Internationale Anmeldenummer:
**PCT/EP2002/005570**

(87) Internationale Veröffentlichungsnummer:
**WO 2002/094949 (28.11.2002 Gazette 2002/48)**

(54) **NACHWEISVERFAHREN ZUR IDENTIFIZIERUNG VON HYDROLASEN**

DETECTION METHOD FOR IDENTIFYING HYDROLASES

PROCEDE DE DETECTION POUR IDENTIFIER DES HYDROLASES

(84) Benannte Vertragsstaaten:
**AT BE CH CY DE DK ES FI FR GB GR IE IT LI LU MC NL PT SE TR**

(30) Priorität: **21.05.2001 DE 10124799**

(43) Veröffentlichungstag der Anmeldung:
**10.03.2004 Patentblatt 2004/11**

(73) Patentinhaber: **BASF Aktiengesellschaft
67056 Ludwigshafen (DE)**

(72) Erfinder:
- **BORNSCHEUER, Uwe, T.
17489 Greifswald (DE)**
- **BAUMANN, Marcus
73669 Lichtenwald (DE)**

(74) Vertreter: **Reitstötter - Kinzebach Patentanwälte,
Sternwartstrasse 4
81679 München (DE)**

(56) Entgegenhaltungen:
**EP-A- 0 089 210 US-A- 4 035 239**

- **BAUMANN M ET AL: "Rapid screening of hydrolases for the enantioselective conversion of 'difficult-to-resolve' substrates" TETRAHEDRON: ASYMMETRY, ELSEVIER SCIENCE PUBLISHERS, AMSTERDAM, NL, Bd. 11, Nr. 23, 1. Dezember 2000 (2000-12-01), Seiten 4781-4790, XP004313852 ISSN: 0957-4166 in der Anmeldung erwähnt**
- **LIU A M F ET AL: "Mapping the substrate selectivity of new hydrolases using colorimetric screening: lipases from Bacillus thermocatenulatus and Ophiostoma piliferum, esterases from Pseudomonas fluorescens and Streptomyces diastatochromogenes" TETRAHEDRON: ASYMMETRY, ELSEVIER SCIENCE PUBLISHERS, AMSTERDAM, NL, Bd. 12, Nr. 4, 19. März 2001 (2001-03-19), Seiten 545-556, XP004234152 ISSN: 0957-4166 in der Anmeldung erwähnt**
- **DAHL A R ET AL: "Carboxylesterases in the respiratory tracts of rabbits, rats and Syrian hamsters." TOXICOLOGY LETTERS. NETHERLANDS APR 1987, Bd. 36, Nr. 2, April 1987 (1987-04), Seiten 129-136, XP008014047 ISSN: 0378-4274**
- **DATABASE WPI Section Ch, Week 198407 Derwent Publications Ltd., London, GB; Class D16, AN 1984-040219 XP002232356 & JP 59 002699 A (NIPPON OILS & FATS CO LTD), 9. Januar 1984 (1984-01-09)**
- **TOMITA KOSUKE ET AL: "Enzymatic determination of acetylcarnitine for diagnostic applications." JOURNAL OF PHARMACEUTICAL AND BIOMEDICAL ANALYSIS, Bd. 24, Nr. 5-6, März 2001 (2001-03), Seiten 1147-1150, XP001148331 ISSN: 0731-7085**
- **KAKLIJ G S ET AL: "MECHANISM OF DEACETYLATION OF N-A ACETYLATED PROTEINS" INDIAN JOURNAL OF BIOCHEMISTRY AND BIOPHYSICS, Bd. 24, Nr. 3, 1987, Seiten 124-128, XP008014037 ISSN: 0301-1208**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 395 673 B1

- DATABASE WPI Section Ch, Week 198409 Derwent Publications Ltd., London, GB; Class D13, AN 1984-052606 XP002232357 & JP 59 011199 A (UNITIKA LTD), 20. Januar 1984 (1984-01-20)

## Beschreibung

Die vorliegende Erfindung betrifft ein Nachweis- oder Screening-Verfahren zur Identifizierung von Hydrolasen, welches insbesondere für das High-Throughput-Screening (HTS) prokaryotischer oder eukaryotischer Proben geeignet ist; Testkits zur Durchführung des Verfahrens; Verfahren zur Isolierung von Hydrolasen mit verändertem Eigenschaftsprofil, wie z.B. mit verbesserter Enantioselektivität, unter Verwendung dieses Nachweisverfahrens.

Stand der Technik:

Hydrolytische Enzyme sind vielseitige Biokatalysatoren und finden vermehrt Anwendung in der organischen Synthese [1]. Innerhalb dieser Enzymklasse werden Lipasen und Esterasen häufig verwendet, da sie eine breite Palette nicht-natürlicher Substrate umsetzen, gewöhnlich sehrstabil in organischen Lösungsmitteln sind und gute bis ausgezeichnete Stereoselektivität bei der kinetischen Auflösung von Racematen oder bei der Desymmetrisierung von prostereogenen Verbindungen zeigen. Aufgrund der enormen Fortschritte auf dem Gebiet des Genetic Engineering nimmt die Zahl der verfügbaren Biokatalysatoren rapide zu. Dies erschwert jedoch die Identifizierung geeigneter Enzyme mithilfe von Reaktionen im Labormaßstab, gefolgt von der Bestimmung der optischen Reinheit mithilfe von Gas-Chromatographie oder HPLC. Außerdem erfordern die kürzlich entwickelten Techniken zur gerichteten (molekularen) Evolution [2] höchst anspruchsvolle Tests zum High-Throughput-Screening (HTS) großer Bibliotheken, welche gewöhnlich $10^4$ - $10^8$ Mutanten enthalten.

Der Nachweis von Verbindungen über enzymatische Umsetzungen ist im Prinzip bekannt. Beispielsweise beschreiben Tomita et al. zur diagnostischen Bestimmung von Acetylcamitin einen Enzymassay, bei dem Acetylcamitin unter Bildung von Acetat enzymatisch hydrolysiert wird [17]. Über weitere gekoppelte Enzymreaktionen führt Acetat letztlich unter Verbrauch von $NADH^+$ zur Bildung von Lactat, wobei die Abnahme von $NADH^+$ für die Bestimmung der ursprünglich freigesetzten Menge an Acetat und somit auch der ursprünglich vorhandenen Menge an Acetylcamitin verwendet wird.

Für eine rasche Bestimmung der Enantioselektivität E (auch bezeichnet als das Enantiomeren-Verhältnis) [3] wurden mehrere Testsysteme entwickelt. Kazlauskas et al. bestimmen beispielsweise die Anfangsgeschwindigkeit der Hydrolase-katalysierten Hydrolyse von p-Nitrophenyl-Derivaten reiner chiraler Carbonsäuren ("Quick E") in Gegenwart von Resorufin-Tetradecanoat, zur Erzeugung einer Konkurrenz-Reaktion, um exaktere E-Werte zu erhalten [4]. In ähnlicher Weise werden von Reetz et al. p-Nitrophenyt-Derivate verwendet, um Lipase-Mutanten mit verbesserter Enantioselektivität zu screenen [5]. Probleme aufgrund einer Hintergrund-Absorption in Gegenwart von Kulturüberständen und Auto-Hydrolyse der p-Nitrophenylester wurden durch Anwendung eines Resorufin-basierenden Fluoreszenztests zur Bestimmung von Mutanten einer Esterase aus *Pseudomonas fluorescens* mit erhöhter Enantioselektivität gegenüber 3-Phenylbuttersäure-Derivaten umgangen [6]. Ein anderer fluorogener Test verwendet Umbelliferon-Derivate, welche nach Hydrolase-Spaltung mit Periodat und Rinderserum-Albumin umgesetzt werden [7].

Ein Nachteil all dieser Testsysteme besteht in der Verwendung sperriger chromophorer Gruppen, welche sich gewöhnlich beträchtlich vom "wahren Substrat", das heißt Acetat, unterscheiden. Folglich besteht die Gefahr, dass die "falschen" Mutanten gescreent werden. Um dieses Problem zu umgehen, wurde von Kazlauskas et al. ein Test beschrieben, in dem sowohl Acetate chiraler Alkohole als auch Ester chiraler Carbonsäuren in Gegenwart von p-Nitrophenol als pH-Indikator hydrolysiert werden [8]. Ein stufenweiser Test für das Screening auf enantionselektive Hydrolasen wurde kürzlich von den vorliegenden Erfindem beschrieben [9]. Dabei wird zunächst aus einer Vielzahl von Enzymen jeweils ein Enzym in eine Vertiefung einer Mikrotiterplatte gegeben und dessen Befähigung zur Hydrolyse zugegebener Acetat- oder Butyratsubstrate durch Farbumschlag eines zugegebenen pH-Indikators (Bromthymolblau) nach erfolgter Umsetzung nachgewiesen. Die Enantioselektivität von Enzymen, deren generelle Hydrolyse-Aktivität durch den Farbumschlag gezeigt wurde, wird anschließend durch Gaschromatographie unter Verwendung chiraler Säulen ermittelt. Eine weitere, jedoch nicht quantifizierbare Methode basiert auf der zeitabhängigen IR-thermographischen Bestimmung der Enantioselektivität [10]. Eine relativ genaue Bestimmung wahrer E-Werte kann unter Verwendung von "Pseudo"-Enantiomeren erzielt werden, wobei eine Form isotopenmarkiert wird, wie z.B. deuteriertes Acetat, gefolgt von einer massenspektroskopischen Analyse [11]. Besondere Nachteile dieses Verfahrens sind in der Verwendung reiner deuterierter Enantiomere und in den hohen Ausstattungskosten zu sehen. Kürzlich wurde ein "Super-HTS" beschrieben, bei dem man unter Verwendung einer parallelen Kapillar-Elektrophorese mit chiralen Cyclodextrinen die optische Reinheit von FITCmarkierten Aminen bestimmt hat [12]. Dieses System ist jedoch auf Amine beschränkt und erfordert ebenfalls eine teure Austattüng.

Kurze Beschreibung der Erfindung:

Es ist deshalb Aufgabe der vorliegenden Erfindung, ein schnelles, kostengünstiges Verfahren zum Aktivitätsnachweis von Hydrolasen, wie insbesondere Lipasen und Esterasen, zur Bestimmung der Enantioselektivität von Hydrolasen oder zum Nachweis von Hydrolasen mit verändertem Eigenschaftsprofil bereit zu stellen. Dieses Verfahren soll insbesondere für das HTS von Mikroorganismen-Bibliotheken, wie beispielsweise solche, die durch gerichtete Evolution hergestellt wurden, geeignet sein.

Obige Aufgabe wird gelöst durch Bereitstellung eines Testsystems, welches schnell und kostengünstig in Mikrotiterplatten durchführbar ist und auf einer gekoppelten enzymatischen Umsetzung beruht. Das erfindungsgemäße Testsystem ermöglicht insbesondere die schnelle und zuverlässige Bestimmung der Hydrolase-Aktivität und Enantioselektivität.

Ein erster Gegenstand der Erfindung betrifft ein Nachweis- bzw. Screening-Verfahren für Hydrolasen, wie z.B. enantioselektive oder stereoselektive Hydrolasen, das dadurch gekennzeichnet ist, dass man

a) ein Reaktionsmedium, enthaltend als Substrat wenigstens einen Ester von Essigsäure mit einem chiralen oder prochiralen Alkohol, mit einem Analyten, in dem man die Hydrolase-Aktivität vermutet, unter Essigsäure freisetzenden Bedingungen inkubiert; und

b) die Bildung von Essigsäure mit Hilfe eines gekoppelten enzymatischen Tests nachweist, wobei die gebildete Essigsäure unter stöchiometrischer Bildung von NADH verbraucht wird.

Für den erfindungsgemäß bevorzugten Nachweis enantioselektiver oder stereoselektiver Hydrolasen wird ein Substrat verwendet, welches wenigstens einen Ester von Essigsäure mit einem chiralen oder prochiralen Alkohol umfasst.

Ein weiterer Gegenstand der Erfindung betrifft einen Testkit zur Durchführung des erfindungsgemäßen Screening-Verfahrens, wobei der Testkit neben üblichen Bestandteile $NAD^+$ und wenigstens ein chirales oder prochirales Substrat für die Hydrolase umfasst.

Ein anderer Gegenstand der Erfindung betrifft ein Verfahren zur Isolierung von natürlichen oder künstlichen Hydrolase-Mutanten oder Hydrolase-Varianten mit verändertem Eigenschaftsprofil, das dadurch gekennzeichnet ist, dass man

a) aus einem Prokaryoten oder Eukaryoten wenigstens einen Analyten herstellt;

b) den Analyten mit Hilfe eines erfindungsgemäßen Nachweisverfahrens auf Hydrolase-Aktivität untersucht;

c) im Falle eines positiven Nachweises von Hydrolase-Aktivität im Analyten das Eigenschaftsprofil der Mutante oder Variante bestimmt und mit dem Eigenschaftsprofil einer Referenzprobe vergleicht; und

d) bei Nachweis von im Vergleich zur Referenzprobe verändertem Eigenschaftsprofil, die Mutante oder Variante isoliert.

Die mit Hilfe eines erfindungsgemäßen Verfahrens isolierten Hydrolasen mit verbesserter Enantioselektivität sind dann zur Herstellung chiraler Ester und Alkohole verwendbar.

<u>Figurenbeschreibung:</u>

Die Erfindung wird in den folgenden Abschnitten unter Bezugnahme auf die beiliegenden Schemata und Figuren beschrieben. Dabei zeigt:

**Schema 1** die Hydrolase-katalysierte Reaktion, welche Essigsäure freisetzt. Diese wird durch Adenosin-5'-triphosphat (ATP) und Coenzym A (CoA) zu Acetyl-CoA in Gegenwart von Acetyl-CoA-Synthetase (ACS) umgewandelt. Citrat Synthase (CS) katalysiert die Reaktion zwischen Acetyl-CoA und Oxalacetat unter der Bildung von Citrat. Das für diese Reaktion erforderliche Oxalacetat wird aus L-Malat und Nicotinamid-Adenin-Dinucleotid ($NAD^+$) in Gegenwart von L-Malat Dehydrogenase (L-MDH) gebildet. Die Anfangsgeschwindigkeit der Essigsäurebildung kann somit über die Zunahme der Extinktion bei 340nm aufgrund der Zunahme der NADH-Konzentration ermittelt werden. Bei Verwendung enantiomerenreiner Acetate ist eine Bestimmung der scheinbaren (bzw. "apparenten") Enantioselektivität $E_{app}$ möglich;

**Schema 2** die Modellreaktion für die Evaluierung des gekoppelten enzymatischen Acetattests. Unter den angegebenen Bedingungen zeigt die Esterase aus *Pseudomonas fluorescens* eine hohe (R)-Selektivität (E = 34);

**Schema 3** die Acetate sekundärer Alkohole, welche für die Evaluierung des Tests eingesetzt wurden. 1: alpha-Phenylethytacetat; 2: 2-Acetoxy-but-3-in; 3:1-Methoxy-2-propytacetat; 4: 3-Acetoxy-tetrahydrofuran; 5: Pantolactonacetat;

**Figur 1** die annähernd lineare Zunahme der NADH-Konzentration in Abhängigkeit von der Zeit, beobachtet bei der

Umsetzung von freigesetzter Essigsäure während der PFE-katalysierten Hydrolyse von (R,S)-1. Eine Autohydrolyse wurde in Abwesenheit der Esterase nicht beobachtet;

**Figur 2** den Zusammenhang zwischen Absorption bei 340 nm und (A) Menge des Substrats (R,S)-1 und (B) zunehmender Esterase-Konzentration:

Figur 3 das Ergebnis des erfindungsgemäßen Testsystems bei Hydrolyse der Acetate (R,S)-2 bis 5 unter Verwendung der Lipase B aus *Candida antarctica* (CAL-B);

**Figur 4** die Anfangsgeschwindigkeiten bestimmt unter Verwendung der optisch reinen (R)- bzw. (S)-Enantiomere von (R,S)-1; die Reaktionen wurden unter Verwendung von lyophilisierter PFE (A) oder unter Verwendung von Kulturüberstand, enthaltend PFE durchgeführt (B); dabei ist die halbe Gesamtaktivität gemäß Figur 4 B zu beachten.

<u>Detaillierte Beschreibung der Erfindung:</u>

Die erfindungsgemäße Nachweisreaktion ist grundsätzlich auf bekannte und bisher nicht beschriebene, natürlich vorkommende Hydrolasen oder Mutanten und Varianten solcher Hydrolasen anwendbar. Die natürlichen Hydrolasen können dabei eukaryotischen, insbesondere pflanzlichen oder tierischen, oder prokaryotischen Ursprungs sein.

Eine Hydrolase mit "verändertem Eigenschaftsprofil", welche mit Hilfe des erfindungsgemäßen Verfahrens in besonders einfacher Weise nachweisbar ist, zeigt wenigsten eine der folgenden Eigenschaften: veränderte, insbesondere erhöhte, Enzymaktivität, veränderte, insbesondere erhöhte, Enantioselektivität, veränderte, insbesondere erhöhte, Temperaturstabilität; und veränderte, insbesondere erhöhte, Stabilität in wässriger, organischen und/oder wässrig/organischen Flüssigkeiten; jeweils im Vergleich zu einer Referenz-Hydrolase mit nicht verändertem Eigenschaftsprofil, wie z.B. einer natürlich vorkommenden Hydrolase.

Eine "enantioselektive Hydrolase" im Sinne der Erfindung ist eine Hydrolase, welche bei der hydrolytischen Spaltung eines chiralen oder prochiralen Carbonsäureesters, insbesonders eines Esters einer achiralen Carbonsäure, insbesondere Essigsäure, und eines chiralen oder prochiralen, z.B. sekundären, Alkohols, ein Enantiomer oder eine prostereogene Form bevorzugt umsetzt. Bevorzugt sollte wenigstens eines der fünf erfindungsgemäßen Referenzsubstrate (Schema 3) enantioselektiv umgesetzt werden können.

Das erfindungsgemäße "Screening-Verfahren" umfasst sowohl Einzelmessungen als auch Messreihen mit grundsätzlich unbegrenzter Anzahl von Einzelmessungen.

Als erfindungsgemäßer "Analyt" sind alle Proben umfasst, in welchen man Hydrolase-Aktivität zumindest vermutet. Der Analyt kann eine Hydrolase, gelöst in reiner Form oder im Gemisch mit anderen, z.B. zellulären, Material enthalten, wobei die Hydrolase-Aktivitätdurch die übrigen Bestandteile nicht unterbunden werden soll. Der Analyt kann zum Beispiel ein Zellrohextrakt aus prokaryotischen Zellen, eukaryotischen Zellen, Organen oder Geweben oder ein Kulturüberstand einer eukaryotischen oder prokaryotischen Zellkultur sein.

Ein erfindungsgemäßer"Testkit" umfasst in einem oder mehreren getrennten Teilen die einzelnen Nachweis-bzw. Testreagenzien sowie ggf. dafür geeignete Testgefäße.

Das erfindungsgemäße Testsystem für die schnelle und zuverlässige Detektion von Hydrolase-Aktivität und insbesondere für die Bestimmung ihrer Enantioselektivität über die Bildung von NADH in einer gekoppelten enzymatischen Umwandlung von gebildeter Essigsäure erfüllt überraschenderweise die folgenden wesentlichen Kriterien:

- Stöchiometrischer Zusammenhang zwischen Essigsäurebildung durch Esterhydrolyse und NADH-Bildung.
- Die Hydrolase-katalysierte Essigsäure-Freisetzung ist der geschwindigkeitsbestimmende Schritt.
- Die Zunahme der Extinktion ist über einen großen NADH-Konzentrationsbereich linear.
- Die Hydrolase hat keinen Einfluss auf die folgende Umsetzung der Essigsäure und auf die Komponenten des Testkits (und umgekehrt).
- Der Test ermöglicht eine genaue Bestimmung der Enantioselektivität, um verbesserte Hydrolasen identifizieren zu können.
- Die Acetate sind unter leicht basischen Bedingungen (pH 8,1) während der Reaktion stabil.
- Die "apparenten" E-Werte ($E_{app}$), die aus dem Verhältnis der Reaktionsgeschwindigkeiten der reinen Enantiomere ermittelt werden, stimmen mit den wahren E-Werten ($E_{true}$), bestimmt in Konkurrenz-Experimenten, überein. So erhält man beispielsweise bei Ver wendung von optisch reinem (R)- oder (S)-Acetat von alpha-Phenylethanol in getrennten Vertiefungen von Mikrotiterplatten mit rekombinanter Esterase aus *Pseudomonas fluorescens* (PFE) Werte für die apparente Enantioselektivität $E_{app}$. Lyophylisierte PFE aus Zellextrakten ergab einen $E_{app}$-Wert von 30. Dieser stimmt in etwa mit dem fürdie Hydrolyse eines racemischen Gemisches ermittelten Wert überein ($E_{true}$ = 34), welcher durch gaschromatographische Analyse bestimmt wurde.

- Der Test ist auf Reaktionen in Gegenwart von Zell-Rohextrakt und Kulturüberstände anwendbar, obwohl hierbei ein abweichender E-Wert ermittelt werden kann (für obiges System $E_{app}$ =21).

Zunächst wird im erfindungsgemäßen Testsystem Acetat durch die Hydrolase-katalysierte Hydrolyse eines chiralen Esters freigesetzt und anschließend über eine Kaskade enzymatischer Reaktionen bestimmt [13].

Insbesondere wird in diesem Screening-Verfahren der Essigsäure-Nachweis mit Hilfe eines gekoppelten enzymatischen Tests durchgeführt, welcher bevorzugt ein optischer Test ist.

Die Bestimmung der Essigsäure basiert vorzugsweise auf einem bereits allgemein verfügbaren, kostengünstigen Testkit, welcher ursprünglich für die Nahrungsanalyse entwickelt wurde. Der Testkit besteht im wesentlichen aus den Enzymen Acetyl-CoA Synthetase, Citratsynthase und L-Malat-Dehydrogenase. Er umfasst weiterhin die Substrate und Cosubstrate L-Malat, ATP, $NAD^+$, Coenzym A (CoA-SH), sowie Puffer und Stabilisatoren und gegebenenfalls Magnesiumchlorid. Das bei der hydrolytischen Reaktion freigesetzte Acetat wird in diesem Testsystem stöchiometrisch umgewandelt und bewirkt eine Veränderung in der Absorption (Extinktion) aufgrund der Reduktion von $NAD^+$ zu NADH. Diese Veränderung kann durch spektrophotometrische Messung bei 340 nm, 334 nm oder 365 nm bestimmt werden. Die spektrophotometrische Messung erlaubt insbesondere die Bestimmung der Anfangsgeschwindigkeit für die Bildung von NADH. Aufgrund des stöchiometrischen Zusammenhangs zwischen Essigsäure-Freisetzung und NADH-Bildung kann die Hydrolase-Aktivität bestimmt werden.

Als Analyt können in dem erfindungsgemäßen Verfahren Reinsubstanzen der Hydrolasen aber insbesondere auch Zellrohextrakte oder ein Kulturüberstand eines kultivierten Mikroorganismus eingesetzt werden.

Vorzugsweise erfolgt die gesamte Nachweisreaktion (Vermischen von Analyt und Enzymen und Substraten für Nachweisreaktion und optische Auswertung des Reaktionsansatzes) in Mikrotiterplatten. Dabei wird die durch Esterhydrolyse des eingesetzten chiralen Esters (optische rein oder Racemat) gebildete Essigsäure unter stöchiometrischer Bildung von NADH verbraucht und die NADH-Bildung optisch bei geeigneter Meßwellenlänge, z.B. 340 nm, in jeder Vertiefung der Mikrotiterplatte bestimmt.

In dem erfindungsgemäßen gekoppelten enzymatischen Test erfolgt die stöchiometrische Bildung von NADH dadurch, dass man Essigsäure enzymatisch (insbesondere katalysiert durch Acetyl-CoA Synthetase unter Verbrauch von ATP und Coenzym A) in Acetyl-CoA überführt, welches in einer enzymkatatysierten Reaktion mit Hilfe von Citrat Synthase stöchiometrisch mit Oxalacetat zu Citrat umgesetzt wird. Das hierzu erforderliche Oxalacetat wird enzymatisch mit Hilfe von L-Malat-Dehydrogenase aus L-Malat in Gegenwart von $NAD^+$ unter NADH-Freisetzung gebildet (vgl. Schema 1). Die Reaktion erfolgt bevorzugt in gepuffertem (pH etwa 7,5 bis 8,5, insbesondere pH 8,1) Medium in Gegenwart geeigneter weiterer Hilfsstoffe wie Magnesiumchlorid und gegebenenfalls Enzymstabilisatoren.

Das erfindungsgemäße Verfahren ist insbesondere anwendbar auf Hydrolasen, welche zur Spaltung von Carbonsäureestern, wie zB. Essigsäureestem, befähigt sind. Solche Hydrolasen sind insbesondere ausgewählt unter Esterasen (E.C.3.1.1.1), Lipasen (E.C.3.1.1.3), Amidasen (E.C.3.5.1.4), Acylasen (E.C.3.5.x.y) und Proteasen (E.C.3.4.x.y).

Beispiele für erfindungsgemäß einsetzbare Substrate sind alpha-Phenylethylacetat, 2-Acetoxy but-3-ynyl (2-Acetoxy-3-butin), 1-Methoxy-2-propylacetat, 3-Acetoxy-tetrahydrofuran und Pantolactonacetat, jeweils in optisch reiner Form oder als deren Gemisch, insbesondere Racernat.

In einer besonders bevorzugten Ausführungsform wird das erfindungsgemäße Screening-Verfahren so durchgeführt, dass die Hydrolase-katalysierte Acetat-Bildung der geschwindigkeitsbestimmende Schritt des gesamten Nachweisverfahrens ist.

Ein besonders bevorzugtes Anwendungsgebiet des erfindungsgemäßen Nachweisverfahrens ist ein HTS-Verfahren zur Bestimmung der Hydrolase-Aktivität und/oder-Selektivität in Extrakten oder Kulturüberständen natürlicher oder genetisch modifizierter Mikroorganismen, bei dem eine Vielzahl von Proben systematisch auf Aktivität bzw. verbesserte Enantioselektivität einer bestimmten Hydrolase untersucht wird.

Eine weitere besondere Ausgestaltung des Screening-Verfahrens besteht darin, dass der Nachweisreaktion eine Kultivierung von einer Vielzahl zu analysierenden Mikroorganismen in den Vertiefungen von Mikrotiterplatten und die Herstellung des Analyten aus den erhaltenen Mikroorganismenkulturen vorausgeht.

Das erfindungsgemäße Screening-Verfahren eignet sich nicht nur zur Bestimmung der Enzymaktivität der Hydrolase im Analyten; sondern auch zur Bestimmung der Enantioselektivität für die Hydrolase im Analyten.
Eine spezielle Ausführungsform des erfindungsgemäßen Verfahrens zur Isolierung von natürlichen oder künstlichen Hydrolase-Mutanten oder Hydrolase-Varianten mit erhöhter Enantioselektivität, ist dadurch gekennzeichnet, dass man

a) einen Mikroorganismus, insbesondere einen rekombinanten Mikroorganismus, welche die kodierende Sequenz für eine Hydrolase exprimiert, die durch in vitro-Mutagenese, insbesondere durch Sättigungs-Mutagenese oder gezielte ("directed") Evolution erzeugt wurde und welcher möglicherweise Hydrolase-Aktivität exprimiert, kultiviert;
b) aus der Kultur wenigstens einen Analyten herstellt;
c) den oder die Analyten mit Hilfe eines erfindungsgemäßen Screening-Verfahrens auf Hydrolase-Aktivität untersucht;

d) im Falle eines positiven Nachweises von Hydrolase-Aktivität im Analyten die Enantioselektivität bestimmt und den ermittelten Wert mit dem Wert einer Referenzprobe vergleicht, und

e) bei Nachweis von im Vergleich zur Referenzprobe erhöhten Enantioselektivität das Enzym, gegebenenfalls nach weiterer Kultivierung des Mikroorganismus, aus der Kultur isoliert.

In analoger Weise sind Hydrolasen mit verbesserter Aktivität, Stabilität gegenüber Temperatur und/oder organischen Lösungsmitteln und/oderverbesserter Stereoselektivität nachweisbar und isolierbar.

Evaluierung des Testsystems:

Um zu verifizieren, ob das erfindungsgemäße Testsystem die obigen Kriterien erfüllt, wurde als Modellreaktion (Schema 2) die Hydrolyse von (R,S)-alpha-Phenylethylacetat (auch bezeichnet als (R,S)-1) durch eine rekombinante Esterase aus *Pseudomonas fluorescens* (PFE) verwendet. Frühere Ergebnisse hatten bereits gezeigt, dass PFE dieses Substrat mit hoher Enantioselektivität bei der Hydrolyse sowie bei. Acylierungsreaktionen in organischen Lösungsmitteln umsetzt [14].

Erste Hydrolyse-Reaktionen wurden im ml-Maßstab unter Verwendung von lyophilisierter PFE (1 mg/ml), 50 mM (R,S)-1 und dem im folgenden beschriebenen Essigsäure-Testkit durchgeführt. Außerdem wurde der Umsatz und der Enanfiomeren-Überschuss durch gaschromatographische Analysen von Proben des Reaktionsgemisches bestimmt (Schema 2). Die Inkubation von (R,S)-1 bei pH 8,1 in Gegenwart des Testkits jedoch in Abwesenheit von PFE führte zu keiner NADH-Bildung. Somit kann eine Auto-Hydrolyse von (R,S)-1 ausgeschlossen werden. Darüber hinaus wurde festgestellt, dass weder die Aktivität noch die Enantioselektivität von PFE durch die Testkit-Komponenten beeinflusst wurden (Ergebnisse von nicht gezeigten Kontrollexperimenten). Die maximal beobachtete Zunahme in der Extinktion bei 340 nm betrug 0,3 pro Minute. Dies scheint die maximale Reaktionsgeschwindigkeit bei dem erfindungsgemäßen gekoppelten Testsystem darzustellen. Für'eine höhere Genauigkeit ist jedoch eine niedrigere Geschwindigkeit empfehlenswert.

Übertragung des Testsystems auf Mikrotiterplatten:

Die Reaktion in Mikrotiterplatten (MTP) ergab eine annähernd lineare Zunahme der Extinktion bei 340 nm über einen Zeitraum von 10 Minuten unter Verwendung einer PFE-Konzentration von 2 mg/ml. Die (R,S)-1-Konzentration wurde im Bereich von 5-50 mg/ml variiert (vergl. Figur 1). Im Gegensatz hierzu verhält sich die Extinktionszunahme pro Minute bei einer Meßwellenlänge von 340nm bei Variation der PFE-Konzentrationen oder der (R,S)-1 Konzentration weniger linear (Figuren 2 A und B).

Figur 2 B zeigt jedoch, dass auch unter bzw. über einer Konzentration von 2 mg/ml PFE ein zuverlässiger Aktivitätsnachweis möglich ist. Sogar bei Konzentrationen von 10 mg/ml PFE und 20 mg/ml (R,S)-1 ist die Enzym-gekoppelte Nachweiskaskade unter Freisetzung von NADH immer noch zweimal so schnell wie die Esterase-Reaktion. Tests bei geringeren Hydrolase- und Substrat-Konzentrationen sind jedoch bevorzug, da sie eine genauere Bestimmung der Reaktionsgeschwindigkeit ermöglichen.

Brauchbarkeit anderer Substrate und Enzyme:

Neben der Modellverbindung 1 ist der Test ebenfalls brauchbar für chirale Ester anderer sekundärer Alkohole (vergl. Schema 3). Für diese Verbindungen wurde Lipase B aus *Candida antarctica* (CAL-B) als Katalysator verwendet [15]. Die gekoppelte Umwandlung der(R,S)-Acetate dieser Verbindungen zeigt abnehmende Reaktionsgeschwindigkeiten in folgender Reihenfolge: 2-Acetoxy-but-3-ynyl 2 > 1-Methoxy-2-propylacetat 3 > 3-Acetoxy-tetrahydrofuran 4 > Pantolactonacetat 5. Diese Beobachtung stimmt mit früheren Ergebnissen überein [9] und belegt, dass das erfindungsgemäße Testsystem auch auf die Aktivitätsbestimmung anderer Hydrolasen anwendbar ist.

Bestimmung der Enantioselektivität:

Die Messung der Anfangsgeschwindigkeiten der Essigsäure-Freisetzung aus den reinen Enantiomeren (R)-1 oder(S)-1 (5 mg/ml) bei einer Esterase-Konzentration von 1,0 mg/ml PFE (lyophilisierter Rohextrakt) ergab fast ausschließlich eine Zunahme in der Extinktion für das (R)-Enantiomer. Dies veranschaulicht die hohe Enantioselektivität von PFE (Figur 4A). Die apparente Enantioselektivität $E_{app}$ betrug 30. Dies entspricht in etwa dem Wert von E=34 gemäß Schema 2. In einem weiteren Ansatz wurde ein PFE-enthaltender Kulturüberstand in zwei Teile aufgeteilt, um sicherzustellen, dass für beide Enantiomere die gleiche Enzymkonzentration verwendet wird. Eine Messung der Anfangsgeschwindigkeit beider Enantiomere ergab, dass Verunreinigungen aus der Kultivierung, wie Zellfragmente oder Medienkomponenten, die Bestimmung der freigesetzten Essigsäure qualitativ beeinflussen (Figur 4 B). Es kann nicht ausgeschlossen werden, dass auch Essigsäure aus der Kultivierung zu dieser Beobachtung beiträgt Die mit der Verwendung von rohem Kulturüberstand verbundenen Einflüsse werden auch durch die verringerte Anfangsgeschwindigkeit und die geringere Enantioselektivität ($E_{app}$=21) reflektiert

Andererseits ist anzumerken, dass die Expression von PFE durch Kultivierung von rekombinantern E. coli in kontrollierter Weise durchgeführt werden kann. Somit kann die Menge an PFE pro Vertiefung relativ genau vorhergesagt werden. Die geringere Genauigkeit des Testsystems unter Verwendung von rohem Kulturüberstand könnte beispielsweise dadurch verbessert werden, dass man einen Reinigungsschritt zwischenschaltet, beispielsweise unter Verwendung einer Mikrotiterplatte, auf welcher exprimierte PFE, basierend auf einem einklonierten Histidin-Tag über Metallionenaffinitätschromatographie gereinigt wird.

Experimenteller Teil

Materialien

Die racemischen Acetate 1-3 wurden in der höchsten Reinheitsstufe käuflich erworben. Optisch reines (R)-1 und (S)-1 sowie die Acetate (R,S)-4-5 wurden aus den entsprechenden Alkoholen mit Essigsäurechlorid in Pyridin unter Verwendung von Standardmethoden hergestellt. Die Lipase B aus *Candida antarctica* (CHIRAZYME ® L-2, CAL-B) wurden von Roche Molecular Biochemicals (Penzberg, Deutschland) bezogen. Die Herstellung der rekombinanten Esterase aus *Pseudomonas fluorescens* (PFE) erfolgte in bekannter Weise [16].

Der Testkit für die Bestimmung von Essigsäure (ursprünglich hergestellt und vertrieben durch Roche Diagnostics, Penzberg, Deutschland) wurde von R-Biopharm GmbH (Darmstadt, Deutschland) bezogen und gemäß Anleitungen des Herstellers verwendet. Die spektrophotometrische Bestimmung der NADH-Konzentration wurde bei 340 nm im ml-Maßstab auf einem Ultrospec 3000 Photometer (Pharmacia Biotech Ltd., Uppsala, Schweden) und im μl-Maßstab unter Verwendung eines Fluorimeters (FLUOstar, BMG LabTechnologies, Offenburg, Deutschland) durchgeführt.

**Berechnungen**

Der Enantiomerenüberschuss (% ee) und die $E_{true}$-Werte wurden nach Chen et al. [3] durch gaschromatographische Analyse unter Verwendung einer Heptakis-(2, 3, 6 -tri-O-methyl)-β-cyclodextrin Säule (CS Chromatographie Service GmbH, Langerwehe, Deutschland) auf einem Gaschromatographen des Typs Fisons Instrument HRGC Mega 2 (ThermoQuest Analytische Systeme GmbH, Egelsbach, Deutschland) bestimmt Die apparenten E-Werte ($E_{app}$) wurden aus dem Verhältnis derAnfangsgeschwindigkeiten der NADH-Bildung, jeweils bestimmt für das (R)- und das (S)-Enantiomere unter Verwendung der mit obigem Testkit ermittelten Daten bestimmt.

Berechnung des Enantiomerenüberschusses (%ee):

$$\%ee = (A-B)/(A+B)^{*}\ 100,$$

mit A, B = Molenbrüche der jeweiligen Enantiomere und A>B

Berechnung des E-Wertes:

$$E= [\ln (1-c)^{*}(1-ee_S)] /[ \ln (1-c) ^{*}(1+ee_S)]$$

mit c= Umsatz, $ee_S$ = Enantiomerenüberschuss des Substrates alternativ bei Kenntnis des Enantiomerenüberschuss des Produktes ($ee_P$):

$$E= [\ln (1-c^{*}(1+ee_P)] /[ \ln (1-c ^{*}(1-ee_P)]$$

Allgemeines Verfahren zur Bestimmung von Essigsäure in einem Mikrotiterplatten-Test

Der Testkit (150 μl) wurde mit PFE (20 μl, 2 mg/ml, wenn nicht anders angegeben) oder mit CAL-B (2 mg/ml) in Lösung vermischt. Die Reaktionen wurden durch Zugabe einer Lösung der Substrate 1-5 (20 μl, bei den in den Figuren angegebenen Konzentrationen) in Natriumphosphatpuffer (10 mM, pH 7,3) gestartet. Mischungen des Testkits mit PufferoderZelllysat von nicht induziertem E. coli DH5a, welcher das Gen für rekombinante Esterase aus *Pseudomonas fluorescens* enthält, dienten als Kontrolle. In ähnlicher Weise wurden die Reaktionsgeschwindigkeiten unter Verwendung optisch reiner Substrate bestimmt.

Esterase-katalysierte Hydrolyse von α-Phenylethylacetat (1)

Zu Natriumphosphatpuffer (500μl, 10mM, pH 7,3) wurde PFE (1 mg/ml) und (R,S)-1 (50 mM) in ein Reaktionsgefäß gegeben. Nach einstündigem Schütteln des Reaktionsgemisches bei 37°C erfolgte eine Extraktion mit Diethylether (500 μl). Die organische Phase wurde abgetrennt, getrocknet und direkt für die GC-Analyse zur Bestimmung des Umsatzes und der Enantioselektivität verwendet. Außerdem wurden Reaktionen in Gegenwart von Testkit-Komponenten durchgeführt, um den Einfluss des leicht basischen pH-Wertes (8,1) auf die Esterase-Performance zu ermitteln.

Expression von *Pseudomonas fluorescens* Esterase in Mikrotiterplatten:

Luria Bertani Medium (200 μl) enthaltend Ampicillin (0,1 mg/ml) pro Vertiefung wurde mit E. coli DH5α inokuliert, welche das Plasmid pJOE2792.1 enthielten, das fürdas Gen von *Pseudomonas fluorescens* Esterase codiert [16]. Die Mikrotiterplatten wurden bei 37 °C (190 Upm) in einem Multitron HT Schüttler (Infors AG, Bottmingen, Schweiz) bis zum Erreichen einer optischen Dichte von 0,3 bei 578 nm geschüttelt. Die Expression der Esterase wurde durch Zugabe von L-Rhamnose-Lösung (20μl, 2%, w/v) induziert. Nach 16 Stunden wurden die Zellen abzentrifugiert (10 Minuten bei 4000 Upm und 4 °C) (Zentrifuge 5810 R, Eppendorf-Netheler-Hinz GmbH, Hamburg, Deutschland). Die Zellen wurden in Natriumphosphatpuffer (150 μl, 10 mM, pH 7,3) resuspendiert und in zwei Gefrier-Auftau-Zyklen aufgeschlossen. Schließlich wurden Zellfragmente abzentrifugiert und die Überstände wurden in Eis aufbewahrt oder mit Natriumphosphatpuffer vor ihrer Verwendung verdünnt.

Der hierin beschriebene Test ist das erste Testsystem, dessen Brauchbarkeit für eine direkte Bestimmung der Hydrolase-Enantioselektivität in Zell-Rohextrakt gezeigt werden konnte. Es besitzt den Vorteil, dass das Testprinzip direkt für das HTS in Mikroorganismen-Bibliotheken einsetzbar ist, wie man sie durch gerichtete Evolution erhält. Die Analysezeit je Testbestimmung der Enantioselektivität beträgt etwa 3-4 Minuten (zusätzlich ca. 1-2 Minuten werden für die Pipettierschritte benötigt). Somit können in einer Mikrotiterplatte mit 96 Vertiefungen etwa 45 Enzyme (2 Vertiefungen je Enzym, 6 Vertiefungen für Kontrollansätze) analysiert werden. Auf diese Weise erhält man 540 E-Werte je Stunde. Somit können etwa 13 000 Mutanten pro Tag gescreent werden. Darüber hinaus ist der erfindungsgemäße Test relativ preiswert, da ein Essigsäure-Testkit für etwa 660 Reaktionen reicht. Das erfindungsgemäße Verfahren ist nicht auf die beispielhaft beschriebenen Esterasen und Lipasen und die Bestimmung ihrer Enantioselektivität beschränkt. Weitere andere Enzymaktivitäten, die eine Freisetzung von Essigsäure bewirken, wie Protease-katalysierte oder Amidase-katalysierte Hydrolysen von Amiden, sollten ebenfalls nach dem erfindungsgemäßen Prinzip analysierbar sein.

Literaturverzeichnis

[1] a) R. N. Patel, *Stereoselective Biocatalysis,* Marcel Dekker, NewYork, 2000; b) K. Faber, *Biotransformations in Organic Chemistry,* Springer, Berlin, 2000; c) U. T. Bomscheuer, R. J. Kazlauskas, *Hydrolases in organic synthesis - regio- and stereoselective biotransformations*, Wiley-VCH, Weinheim, **1999.**

[2] a) U. T. Bomscheuer, M. Pohl, *Curr. Opin. Chem. Biol.* 2001, 5, 137-143; b) U. T. Bornscheuer, *Biocat. Biotransf.* **2001,** in press; c) U. T. Bomscheuer, *Angew. Chem.* **1998**, *110,* 3285-3288; *Angew. Chem. Int Ed. Engl.* **1998**, 37,3105-3108, d) F. H. Amold, A. A. Volkov, *Curr.* Opin. Chem. *Biol.* **1999,** 3, 54-59; e) J. D. Sutherland, *Curr. Opin. Chem. Biol.* **2000**, 4, 263-269; f) M. B. Tobin, C. Gustafsson, G. W. Huisman, *Curr. Opin. Struct. Biol.* **2000**, *10,* 421-427.

[3] C. S. Chen, Y. Fujimoto, G. Girdaukas, C. J. Sih, *J. Am. Chem.* Soc. **1982**, 104, 7294-7299.

[4] L. E. Janes, R. J. Kaziauskas, *J. Org. Chem.* **1997**, 62,4560-4561.

[5] M. T. Reetz, A. Zonta, K. Schimossek, K. Liebeton, K.-E. Jaeger, *Angerv. Chem.* **1997,** *109*, 2961-2963; *Angew. Chem. Int. Ed. Engl.* **1997,** 36, 2830-2832.

[6] E. Henke, U. T. Bomscheuer, *Biol. Chem.* **1999,** 380, 1029-1033.

[7] F. Badalassi, D. Wahler, G. Klein, P. Crotti, J.-L. Reymond, *Angew. Chem.* **2000,** 112, 4233-4236; *Angew. Chem. Int Ed. Engl.* **2000,** 39, 4067-4070.

[8] a) L. E. Janes, A. C. Löwendahl, R. J. Kazlauskas, Chem. Eur. J. 1998,4,2324-2331: b) A. M. F. Liu, N. A. Somers, R. J. Kazlauskas, T. S. Brush, F. Zocher, M. M. Enzelberger, U. T. Bornscheuer, G. P. Horsman, A. Mezzetti, C. Schmidt-Dannert, R. D. Schmid, *Tetrahedron: Asymmetry* 2001, 12, 545-556

[9] M. Baumann, B. Hauer, U. T. Bomscheuer, *Tetrahedron: Asymmetry* 2000, 11, 4781-4790.

[10] M. T. Reetz, M. H. Becker, K. M. Kühling, A. Holzwarth, *Angew. Chem.***1998**, *111,* 2792-2795; *Angew. Chem. Int. Ed Engl.* **1998**, 37, 2647-2650.

[11] M. T. Reetz, M. H. Becker, H. W. Klein, D. Stöckigt, *Angew. Chem.* **1999**, *111,* 1872-1875; *Angew. Chem. Int. Ed. Engl.* **1999***,* 38,1758-1761.

[12] M. T. Reetz, K. M. Kühling, A. Deege, H. Hinrichs, D. Belder*, Angew. Chem.* **2000,** 112, 4049-4052; *Angew. Chem. Int Ed. Engt.* **2000**, 39, 3891-3893.

[13] a) H. U. Bergmeyer (Ed.), *MethodsofEnzymaticAnalysis, Vol.* 1, Wiley-VCH, Weinheim 1974, p. 112-117; b) , H. U. Bergmeyer (Ed.), *Methods of Enzymatic Analysis, Vol. 3,* Wiley-VCH, Weinheim 1974, p. 1520-1528.

[14] N. Krebsfänger, K. Schierholz, U. T. Bomscheuer, *J. Biotechnol.* 60 (1998) 105-111.

[15] E. M. Anderson, K. M. Larsson, O. Kirk, *Biocatal. Biotransform.* **1998,** 16, 181-204.

[16] N. Krebsfänger, F. Zocher, J. Altenbuchner, U. T. Bornscheuer, *Enzyme Microb. Technol. 21* (1998) 641-646.

[17] K. Tomita, S. Sakurada, S. Minami, *J. Pharmaceut. Biomedical Anal.* **2001**, 24, 1147-1150.

## Schema 1

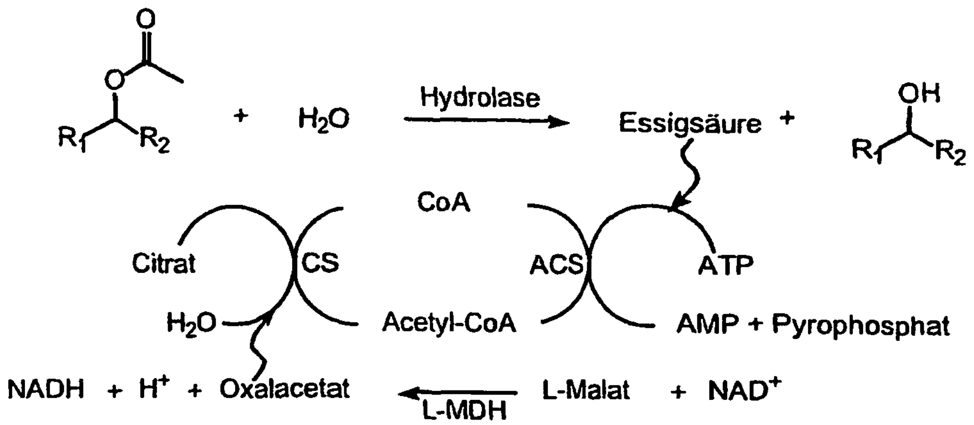


## Schema 2

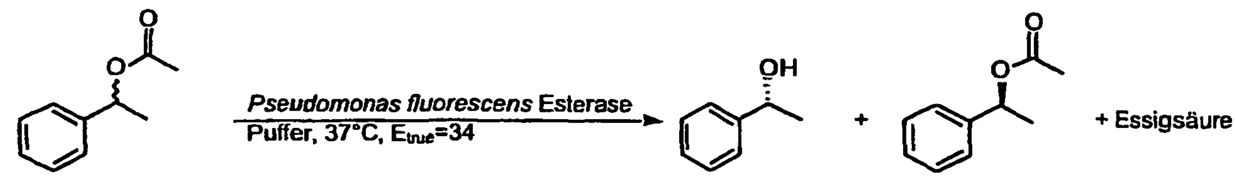


(*R,S*)-α-Phenyl-ethylacetat (*R*)-α-Phenylethanol 93 %ee (*S*)-α-Phenylethyl-acetat, *21* %ee

## Schema 3

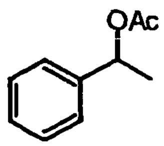
**1**

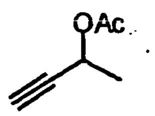
**2**

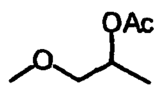
**3**

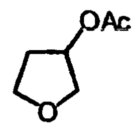
**4**

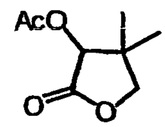
**5**

## Patentansprüche

1. Nachweisverfahren zur Bestimmung der Enantiosetektiviät oder Stereoselektivität von Hydrolasen, **dadurch gekennzeichnet, dass** man

   a) ein Reaktionsmedium, enthaltend als Substrat wenigstens einen Ester von Essigsäure mit einem chiralen oder prochiralen Alkohol, mit einem Analyten, in dem man die Hydrolase-Aktivität vermutet, unter Essigsäure freisetzenden Bedingungen inkubiert; und
   b) die Bildung von Essigsäure mit Hilfe eines gekoppelten enzymatischen Tests nachweist, wobei die gebildete Essigsäure unter stöchiometrischer Bildung von NADH verbraucht wird.

2. Nachweisverfahren nach Anspruch 1, **dadurch gekennzeichnet, dass** der Essigsäure-Nachweis in einem optischen Test erfolgt.

3. Nachweisverfahren nach einem der vorhergehenden Ansprüche, **dadurch gekennzeichnet, dass** der Analyt ein Zellrohextrakt oder ein Kulturüberstand eines kultivierten Mikroorganismus oder kultivierter pflanzlicher oder tierischer Zellen ist oder aus Pflanzen, Tieren oder Organen oder Teilen davon abgeleitet ist.

4. Nachweisverfahren nach einem der vorhergehenden Ansprüche, **dadurch gekennzeichnet, dass** die gesamte Nachweisreaktion in Mikrotiterplatten erfolgt

5. Nachweisverfahren nach Anspruch 1. **dadurch gekennzeichnet, dass** in dem gekoppelten enzymatischen Test die stöchiometrische Bildung von NADH **dadurch** erfolgt, dass man Essigsäure enzymatisch in Acetyl-CoA überführt, welches in einer enzymkatalysierten Reaktion stöchiometrisch mit Oxalacetat zu Citrat umgesetzt wird, wobei Oxalactetat enzymatisch aus L-Malat in Gegenwart von $NAD^+$ unter NADH-Freisetzung gebildet wird.

6. Nachweisverfahren nach einem der vorhergehenden Ansprüche, **dadurch gekennzeichnet, dass** NADH spektrophotometrisch über die Extinktion bei 340nm nachgewiesen wird.

7. Nachweisverfahren nach einem der vorhergehenden Ansprüche, **dadurch gekennzeichnet, dass** die Hydrolase ausgewählt ist unter Esterasen (E.C.3.1.1.1), Lipasen (E.C.3.1.1.3). Amidasen (E.C.3.5.1.4). Acylasen (E.C.3.5.x.y) und Proteasen (E.C.3.4.x.y).

8. Nachweisverfahren nach einem der vorhergehenden Ansprüche, **dadurch gekennzeichnet, dass** das chirale Substrat als racemisches Gemisch oder in optisch reiner Form eingesetzt wird.

9. Nachweisverfahren nach einem der vorhergehenden Ansprüche, **dadurch gekennzeichnet, dass** das Substrat ausgewählt ist unter alpha-Phenylethylacetat, 2-Acetoxy but-3-in 1-Methoxy-2-propylacetat, 3-Acetoxy-tetrahydrofüran und Pantolactonacetat

10. Nachweisverfahren nach einem der vorhergehenden Ansprüche, **dadurch gekennzeichnet, dass** die Hydrolase-katalyslerte Acetat-Bildung der geschwindlgkeitsbestimmende Schritt des Nachweisverfahrens ist.

11. Nachweisverfahren nach einem der vorhergehenden Ansprüche, **dadurch gekennzeichnet, dass** es ein HTS-Verfahren zur Bestimmung der Hydrolase-Aktivitat und/oder -Selektivität in Extrakten oder Kulturüberständen natürlicher oder genetisch modifizierter Mikroorganismen ist.

12. Nachweisverfahren nach einem der vorhergehenden Ansprüche, **dadurch gekennzeichnet, dass** der Nachweisreaktion eine Kultivierung von zu analysierenden Mikroorganismen in Mikrotiterplatten und die Herstellung des Analyten aus der Mikroorganismenkultur vorausgeht.

13. Testkit zur Durchführung eines Nachweisverfahrens nach einem der vorhergehenden Ansprüche, umfassend $NAD^+$ und wenigstens ein chirales oder prochirales Substrat für die Hydrolase.

14. Verfahren zur Isolierung von natürlichen oder künstlichen Hydrolase-Mutanten oder Hydrolase-Varianten mit verändertem Eigenschaftsprofil, **dadurch gekennzeichnet, dass** man

   a) aus einem Prokaryoten oder Eukaryoten einen Analyten herstellt;

b) den Analyten mit Hilfe eines Nachweisverfahrens nach einem der Ansprüche 1 bis 12 auf Hydrolase-Aktivität untersucht;

c) im Falle eines positiven Nachweises von Hydrolase-Aktivität im Analyten das Eigenschaftsprofil der Mutante oder Variante bestimmt und mit dem Eigenschaftsprofil einer Referenzprobe vergleicht und

d) bei Nachweis von im Vergleich zur Referenzprobe verändertem Eigenschaftsprofil die Mutante oder Variante isoliert.

**15.** Verfahren nach Anspruch 14. **dadurch gekennzeichnet, dass** der Mikroorganismus ein rekombinanter Mikroorganismus ist, welcher die kodierende Sequenz für eine Hydrolase exprimiert, die durch in vitro-Mutagenese, insbesondere durch Sättigungs-Mutagenese oder gerichtete ("directed") Evolution erzeugt wurde.

**16.** Verfahren nach Anspruch 14 oder 15, **dadurch gekennzeichnet, dass** das veränderte Eigenschaftsprofil wenigstens eine der folgenden Eigenschaften umfasst: veränderte Enzymaktivität, veränderte Enantioselektivität, veränderte Temperaturstabilität; und veränderte Stabilität in wässrigen, organischen und/oder wässrig/organischen Flüssigkeiten.

## Claims

**1.** A detection method for determining the enantioselectivity or stereoselectivity of hydrolases, which comprises

a) incubating a reaction medium comprising as substrate at least one ester of acetic acid with a chiral or prochiral alcohol, with an analyte in which hydrolase activity is suspected, under acetic acid-liberating conditions; and

b) detecting the formation of acetic acid with the aid of a coupled enzymatic assay, the acetic acid formed being consumed with stoichiometric formation of NADH.

**2.** The detection method according to Claim 1, wherein the acetic acid detection takes place in an optical assay.

**3.** The detection method according to any of the preceding claims, wherein the analyte is a crude cell extract or a culture supernatant of a cultivated microorganism or cultivated plant or animal cells; or is derived from plants, animals or organs or parts thereof.

**4.** The detection method according to any of the preceding claims, wherein the overall detection reaction takes place in microtiter plates.

**5.** The detection method according to claim 1, wherein the stoichiometric formation of NADH takes place in the coupled enzymatic assay through enzymatic conversion of acetic acid into acetyl-CoA which is converted stoichiometrically in an enzyme-catalyzed reaction with oxaloacetate into citrate, with oxaloacetate being formed enzymatically from L-malate in the presence of $NAD^+$ with liberation of NADH.

**6.** The detection method according to any of the preceding claims, wherein NADH is detected spectrophotometrically via the extinction at 340 nm.

**7.** The detection method according to any of the preceding claims, wherein the hydrolase is selected from esterases (E.C.3.1.1.1), lipases (E.C.3.1.1.3), amidases (E.C.3.5.1.4), acylases (E.C.3.5.x.y) and proteases (E.C.3.4..x.y).

**8.** The detection method according to any of the preceding claims, wherein the chiral substrate is employed as racemic mixture or in optically pure form.

**9.** The detection method according to any of the preceding claims, wherein the substrate is selected from alpha-phenylethyl acetate, 2-acetoxy-but-3-yne, 1-methoxy-2-propyl acetate, 3-acetoxytetrahydrofuran and pantolactone acetate.

**10.** The detection method according to any of the preceding claims, wherein the hydrolase-catalyzed acetate formation is the rate-determining step in the detection method.

**11.** The detection method according to any of the preceding claims, which is an HTS method for determining the hydrolase activity and/or selectivity in extracts or culture supernatants of natural or genetically modified microorganisms.

**12.** The detection method according to any of the preceding claims, wherein the detection reaction is preceded by a cultivation of microorganisms to be analyzed in microtiter plates and preparation of the analyte from the microorganism culture.

**13.** A test kit for carrying out a detection method according to any of the preceding claims, comprising $NAD^+$ and at least one chiral or prochiral substrate for the hydrolase.

**14.** A method for isolating natural or artificial hydrolase mutants or hydrolase variants with an altered profile of properties, which comprises

a) preparing an analyte from a prokaryote or eukaryote;
b) investigating the analyte for hydrolase activity with the aid of a detection method according to any of claims 1 to 12;
c) in the event of a positive detection of hydrolase activity in the analyte, determining the profile of properties of the mutant or variant, and comparing with the profile of properties of a reference sample; and
d) if the profile of properties is detected to be altered by comparison with the reference sample, isolating the mutant or variant.

**15.** The method according to claim 14, wherein the microorganism is a recombinant microorganism which expresses the coding sequence for a hydrolase which has been generated by in vitro mutagenesis, in particular by saturation mutagenesis or directed evolution.

**16.** The method according to claim 14 or 15, wherein the altered profile of properties comprises at least one of the following properties: altered enzymic activity, altered enantioselectivity, altered temperature stability; and altered stability in aqueous, organic and/or aqueous/organic liquids.

## Revendications

**1.** Procédé de détection pour déterminer l'énantiosélectivité ou la stéréosélectivité d'hydrolases, **caractérisé en ce que**

a) on met à incuber, dans des conditions libérant de l'acide acétique, un milieu réactionnel contenant, comme substrat, au moins un ester d'acide acétique avec un alcool chiral ou prochiral avec un analyte dans lequel on présume l'activité d'hydrolase, et
b) on détecte la formation d'acide acétique à l'aide d'un test enzymatique couplé, l'acide acétique formé étant consommé avec formation stoechiométrique de NADH.

**2.** Procédé de détection suivant la revendication 1, **caractérisé en ce que** la détection d'acide acétique a lieu dans un test optique.

**3.** Procédé de détection suivant l'une des revendications précédentes, **caractérisé en ce que** l'analyte est un extrait cellulaire brut ou un surnageant de culture d'un micro-organisme cultivé ou de cellules végétales ou animales cultivées ou est dérivé de plantes, d'animaux ou d'organes ou de parties de ceux-ci.

**4.** Procédé de détection suivant l'une des revendications précédentes, **caractérisé en ce que** la réaction de détection globale a lieu dans des plaques de microtitrage.

**5.** Procédé de détection suivant la revendication 1, **caractérisé en ce que**, dans le test enzymatique couplé, la formation stoechiométrique de NADH a lieu par le fait qu'on convertit par voie enzymatique de l'acide acétique en acétyl-CoA qui, dans une réaction catalysée par une enzyme, est amené à réagir de manière stoechiométrique avec de l'oxalacétate pour former du citrate, l'oxalacétate étant formé par voie enzymatique à partir de L-malate en présence de $NAD^+$, avec libération de NADH.

**6.** Procédé de détection suivant l'une des revendications précédentes, **caractérisé en ce que** du NADH est détecté par spectrophotométrie concernant l'extinction à 340 nm.

**7.** Procédé de détection suivant l'une des revendications précédentes, **caractérisé en ce que** l'hydrolase est choisie parmi des estérases (E.C.3.1.1.1), des lipases (E.C.3.1.1.3), des amidases (E.C.3.5.1.4), des acylases (E.C.3.5.x.y) et des protéases (E.C.3.4.x.y).

**8.** Procédé de détection suivant l'une des revendications précédentes, **caractérisé en ce que** le substrat chiral est mis en oeuvre sous la forme d'un mélange racémique ou sous une forme optiquement pure.

**9.** Procédé de détection suivant l'une des revendications précédentes, **caractérisé en ce que** le substrat est choisi parmi de l'acétate d'alphaphényléthyle, du 2-acétoxy-3-butyne, de l'acétate de 1-méthoxy-2-propyle, du 3-acétoxytétrahydrofuranne et de l'acétate de pantolactone.

**10.** Procédé de détection suivant l'une des revendications précédentes, **caractérisé en ce que** la formation d'acétate catalysée par de l'hydrolase est l'étape de détermination de vitesse du procédé de détection.

**11.** Procédé de détection suivant l'une des revendications précédentes, **caractérisé en ce qu'**il est un procédé HTS pour déterminer l'activité et/ou la sélectivité d'hydrolase dans des extraits ou des surnageants de culture de micro-organismes naturels ou génétiquement modifiés.

**12.** Procédé de détection suivant l'une des revendications précédentes, **caractérisé en ce que** la réaction de détection précède une mise en culture de micro-organismes à analyser dans des plaques de microtitrage et la préparation de l'analyte à partir de la culture de micro-organismes.

**13.** Trousse d'essai pour effectuer un procédé de détection suivant l'une des revendications précédentes, comprenant du $NAD^+$ et au moins un substrat chiral ou prochiral pour l'hydrolase.

**14.** Procédé d'isolement de mutants ou variants d'hydrolase naturels ou synthétiques présentant un profil de propriétés modifié, **caractérisé en ce que**

a) on prépare un analyte à partir d'un procaryote ou d'un eucaryote,
b) on examine l'analyte à propos de l'activité d'hydrolase à l'aide d'un procédé de détection suivant l'une des revendications 1 à 12,
c) dans le cas d'une détection positive d'activité d'hydrolase dans l'analyte, on détermine le profil de propriétés des mutants ou variants et on compare avec le profil de propriétés d'un échantillon de référence, et
d) lors de la détection d'un profil de propriétés modifié en comparaison de l'échantillon de référence, on isole les mutants ou variants.

**15.** Procédé suivant la revendication 14, **caractérisé en ce que** le micro-organisme est un micro-organisme recombinant qui exprime la séquence codant pour une hydrolase qui a été produite par mutagénèse in vitro, en particulier par mutagénèse à saturation ou évolution dirigée ("directed").

**16.** Procédé suivant la revendication 14 ou 15, **caractérisé en ce que** le profil de propriétés modifié comporte au moins une des propriétés suivantes: une activité enzymatique modifiée, une énantiosélectivité modifiée, une stabilité thermique modifiée et une stabilité modifiée dans des liquides aqueux, organiques et/ou aqueux/organiques.

Fig.1

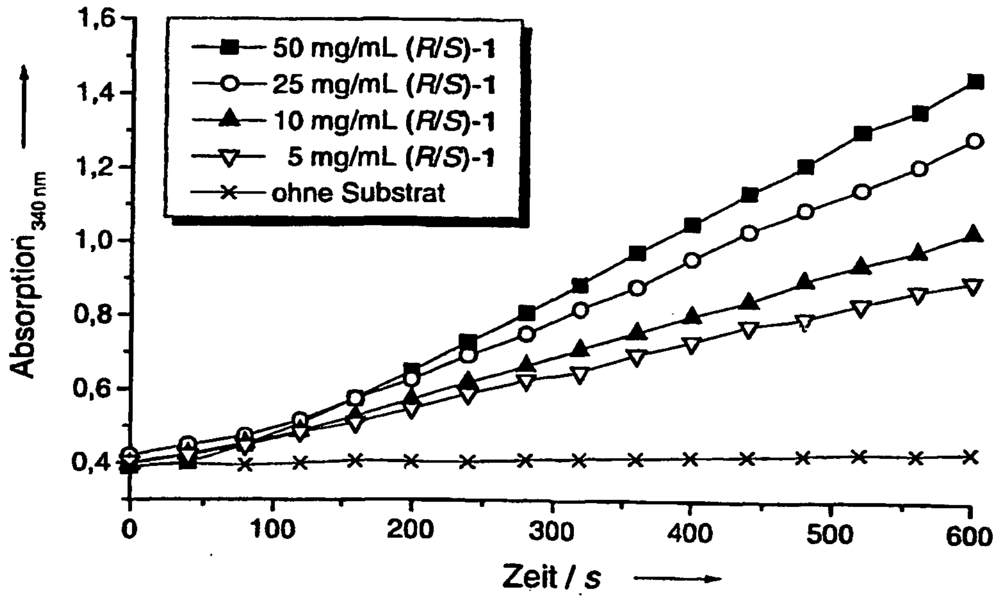
1,6
1,4
1,2
1,0
0,8
0,6
0,4
Absorption 340 nm
50 mg/mL (R/S)-1
25 mg/mL (R/S)-1
10 mg/mL (R/S)-1
5 mg/mL (R/S)-1
ohne Substrat
0
100
200
300
400
500
600
Zeit / s

Fig.2

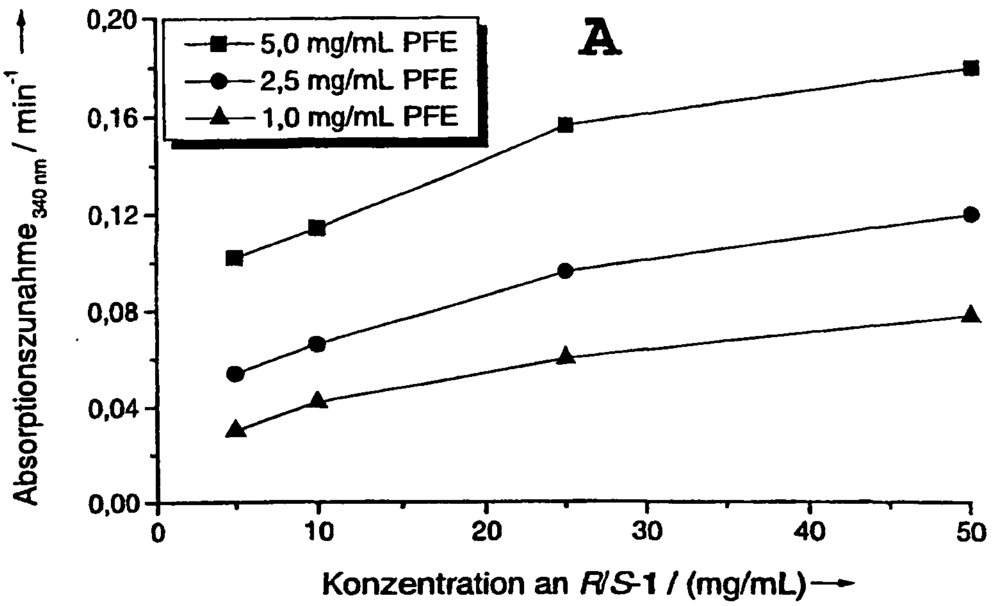
A
5,0 mg/mL PFE
2,5 mg/mL PFE
1,0 mg/mL PFE
Absorptionszunahme$_{340\,nm}$ / min$^{-1}$
0,00
0,04
0,08
0,12
0,16
0,20
0
10
20
30
40
50
Konzentration an R/S-1 / (mg/mL)

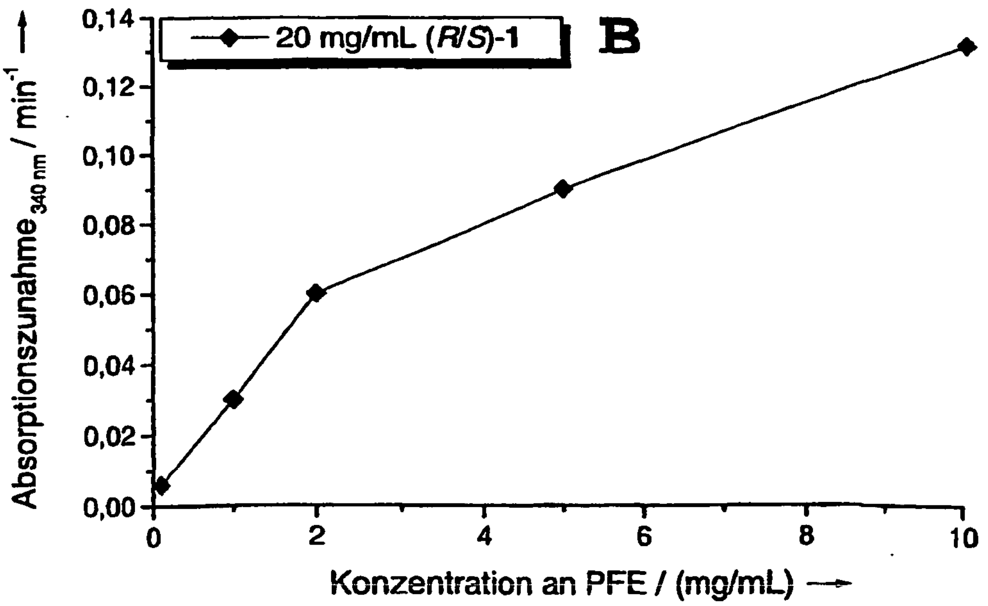
B
20 mg/mL (R/S)-1
Absorptionszunahme$_{340\,nm}$ / min$^{-1}$
0,00
0,02
0,04
0,06
0,08
0,10
0,12
0,14
0
2
4
6
8
10
Konzentration an PFE / (mg/mL)

Fig.3

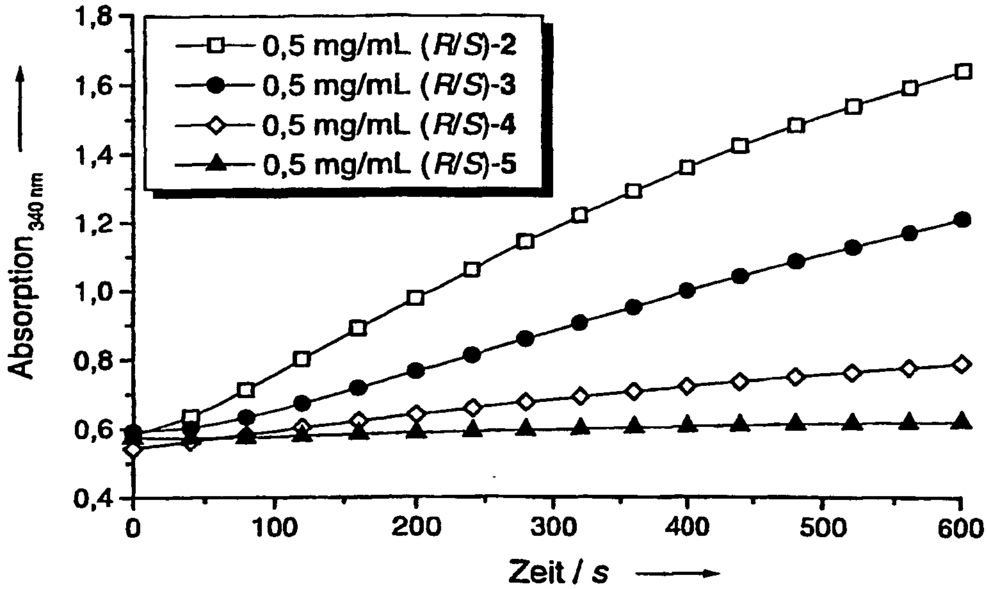
1,8
1,6
1,4
1,2
1,0
0,8
0,6
0,4
Absorption 340 nm
0
100
200
300
400
500
600
Zeit / s
0,5 mg/mL (R/S)-2
0,5 mg/mL (R/S)-3
0,5 mg/mL (R/S)-4
0,5 mg/mL (R/S)-5

Fig.4

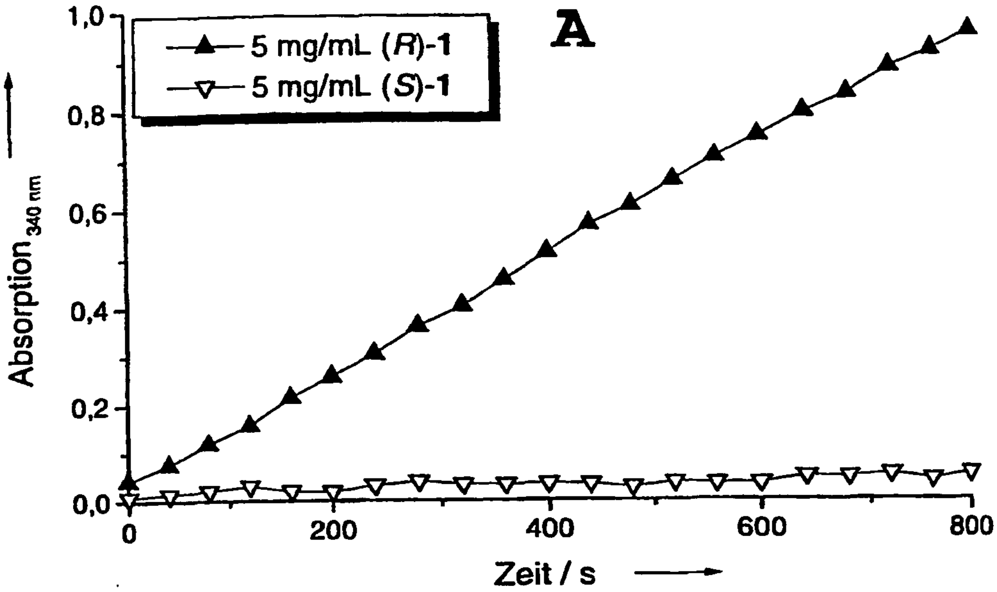
A
5 mg/mL (R)-1
5 mg/mL (S)-1
Absorption 340 nm
1,0
0,8
0,6
0,4
0,2
0,0
0
200
400
600
800
Zeit / s

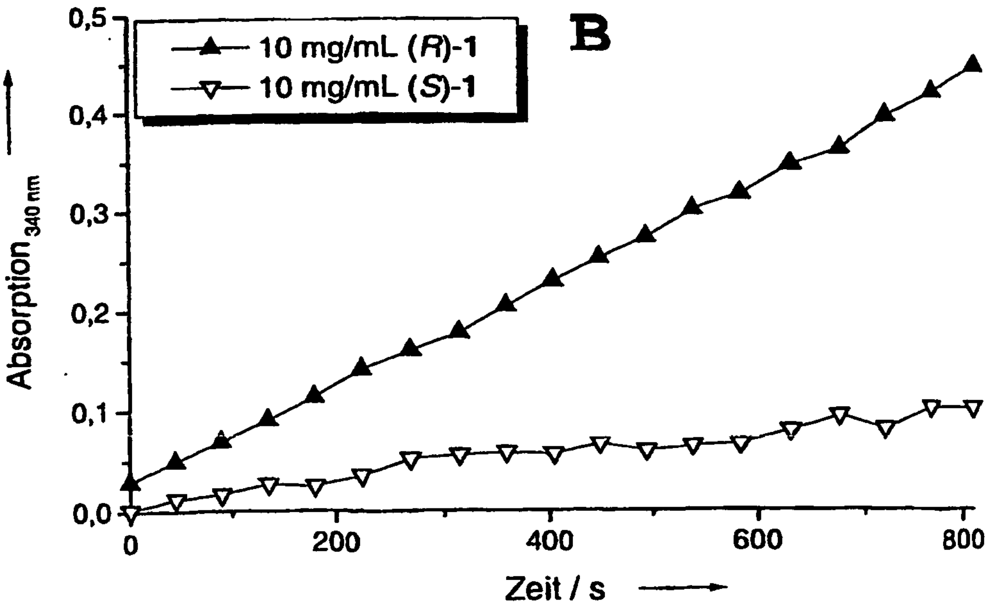
B
10 mg/mL (R)-1
10 mg/mL (S)-1
Absorption 340 nm
0,5
0,4
0,3
0,2
0,1
0,0
0
200
400
600
800
Zeit / s